# EUROPEAN PATENT SPECIFICATION

(11) **EP 3 970 596 B1**
(45) Date of publication and mention of the grant of the patent: **06.08.2025**
(21) Application number: 19928897.8
(22) Date of filing: 21.05.2019
(51) Int. Cl.: A61B 1/00, A61B 1/012, A61B 1/05, A61B 1/06

(54) **ENDOSCOPIC DEVICE**
ENDOSKOPIEVORRICHTUNG
DISPOSITIF ENDOSCOPIQUE

(30) Priority: 13.05.2019 CN 201910392836
(43) Date of publication of application: 23.03.2022
(73) Proprietor: Anqing Medical Co., Ltd, Shanghai 201201 (CN)
(72) Inventor: YAN, Hang, Shanghai 201201 (CN); TANG, Wei, Shanghai 201201 (CN); ZHANG, Along, Shanghai 201201 (CN)
(74) Representative: London IP Ltd
(86) International application number: PCT/CN2019/087774
(87) International publication number: WO 2020/228052

(56) References cited:
- CN-A- 101 371 775
- CN-A- 102 348 404
- CN-A- 107 080 513
- CN-U- 208 725 690
- US-A1- 2002 087 047
- US-A1- 2016 058 383
- US-A1- 2017 135 560
- US-A1- 2017 265 732
- US-A9- 2016 278 614

## Description

### TECHNICAL FIELD

The present invention relates to the field of medical equipment, in particular, to an endoscope device.

### BACKGROUND

In recent years, endoscopes and related surgical instruments have been widely used in the field of minimally invasive diagnosis and treatment. With the rapid development of minimally invasive medical technology, higher requirements have been placed on endoscopes. According to the different parts reached by the endoscope, the endoscope can be classified into neuroscope, urethral cystoscope, resectoscope, laparoscope, arthroscope, sinusoscope, laryngoscope, etc. According to the bending of the head of the endoscope, the endoscope can be divided into flexible endoscope and rigid endoscope. The endoscope structure can be reusable.

The structure of the repeatedly used endoscope is in frequent contact with patients and medical personnel, so it needs to be sterilized and disinfected. However, due to the complicated structure of the endoscope, the image acquisition components, light source components, medical-related equipment, etc. are generally packaged with encapsulating glue to form a package structure. After the encapsulating glue is sterilized by damp heat for many times, its structure is easy to be no longer stable, as a result, the package structure and the endoscope structure are no longer stable, and the damp heat may cause the sterilization effect to become unsatisfactory.

US2017135560 discloses a compound sheath for encasing an endoscope during an endoscopic procedure.

US2016278614 discloses a small diameter endoscopic device for minimally invasive diagnosis and treatment of joint injuries according to the preamble of independent claim 1.

US2002087047A1 discloses a small diameter imaging probe or endoscope, a solid state imaging device, and a light transmitting path that collects light at a distal end of the probe and directs the light along the length of the probe to the imaging device.

### SUMMARY

The present invention provides an endoscope device, in order to solve the problem that the structure is no longer stable and the sterilization effect becomes unsatisfactory in the process of sterilization and disinfection.

According to the present invention as defined in independent claim 1, an endoscope device is provided, comprising
a disposable flexible lens sheath, a reusable endoscope body, and a disposable sterilization jacket, wherein the endoscope body can be received in the flexible lens sheath from an endoscope receiving structure of the flexible lens sheath, and the sterilization jacket is mounted at an entrance of the endoscope receiving structure, isolate the endoscope body received in the flexible lens sheath from the outside;
a light source component is provided near a front end of the flexible lens sheath, the endoscope body is provided with a conductive component, a rear end of the endoscope is connected with a power cord, and the power cord is conducted with the conductive component; after the endoscope body is received in the flexible lens sheath, the conductive component is in direct or indirect conduction with a light source lead of the light source component, so that the light source component is powered;
the sterilization jacket includes a connecting portion, a foldable antibacterial cover and a compression box; a rear end of the connecting portion is connected to one end of the foldable antibacterial cover, and the other end of the foldable antibacterial cover is connected to the compression box; the compression box is disposed at a rear end of the foldable antibacterial cover, and at least part of the foldable antibacterial cover may be folded in the compression box; the foldable antibacterial sheath may be isolated from the rear end of the endoscope body that is connected to the flexible lens sheath after being unfolded.

Optionally, the endoscope body comprises an endoscope handle, an endoscope flexible tube connected to the endoscope handle and an image acquisition package structure disposed at a front end of the endoscope flexible tube, and the conductive component is disposed at the endoscope handle.

Optionally, the image acquisition package structure comprises an image acquisition component and a circuit board that are packaged together, the circuit board is in direct or indirect conduction with the power cord, and the image acquisition component is connected to the circuit board.

Optionally, the flexible lens sheath comprises an lens sheath base, an lens sheath flexible tube and an insertion portion, the lens sheath base is connected to a rear end of the lens sheath flexible tube, and the insertion portion is disposed at a front end of the lens sheath flexible tube;

The endoscope receiving structure is disposed at the lens sheath base, a flexible tube channel for receiving the endoscope flexible tube is formed in the lens sheath flexible tube and the insertion portion, and the flexible tube channel is directly or indirectly communicated with a channel in the endoscope receiving structure.

Optionally, the flexible lens sheath is provided with a multi-lumen tube, the multi-lumen tube is provided with the flexible tube channel for receiving the endoscope flexible tube, and the flexible tube channel is directly or indirectly communicated with the channel in the endoscope receiving structure.

Optionally, the light source lead passes through a light source lead channel;
the light source lead channel is disposed in the multi-lumen tube, and the light source lead channel is isolated from the flexible tube channel, or the light source lead channel is disposed outside the multi-lumen tube.

Optionally, the multi-lumen tube is further provided with an instrument tube channel, the instrument tube channel is isolated from the flexible tube channel, and the instrument tube channel and the flexible tube channel are distributed in a splayed shape.

Optionally, the flexible lens sheath is further provided with an instrument tube receiving structure for receiving an instrument tube, and the instrument tube channel is directly or indirectly communicated with a channel in the instrument tube receiving structure.

Optionally, the flexible lens sheath is provided with at least one of:
a water and gas valve structure;
a bending control structure for controlling the bending of the lens sheath flexible tube;
a locking mechanism for locking the endoscope body and the flexible lens sheath.

In the endoscope device provided in the present invention, with the disposable flexible lens sheath, the reusable endoscope body and the disposables terilization jacket, the relevant personnel will only contact with the disposable flexible lens sheath and sterilization jacket and is completely isolated from the reusable endoscope body when he uses the endoscope device in the present invention, which may effectively avoid the occurrence of bacterial contamination, reduce the risk of surgical infection, and is convenient, safe and reliable to use. Therefore, the present invention may avoid or reduce the sterilization and disinfection of the endoscope body, thereby avoiding or reducing the problems caused by the sterilization and disinfection. In addition, since only the flexible lens sheath and the sterilization jacket are disposable, the costs may be reduced.

And also, in the present invention, since the light source portion is disposed in the flexible lens sheath, the endoscope body may only supply power to the light source portion after being connected to the lens sheath, which may avoid the astigmatism and reflection caused by the integration of the light source inside the endoscope body as compared with the solution in which the image acquisition component and the light source component are packaged together in the endoscope body.

### BRIEF DESCRIPTION OF THE DRAWINGS

To describe the technical solutions in the embodiments of the present invention or in the prior art more clearly, accompanying drawings required to describe the embodiments or the prior art are briefly described below. It is obvious that the accompanying drawings described below are only some embodiments of the present invention. It is apparent to those skilled in the art that other drawings may be further obtained based on the accompanying drawings without inventive effort.
Fig. 1 is a schematic structural diagram 1 of an endoscope device according to an embodiment of the present invention;
Fig. 2 is a schematic structural diagram 2 of an endoscope device according to an embodiment of the present invention;
Fig. 3 is a schematic structural diagram of an endoscope body according to an embodiment of the present invention;
Fig. 4 is a cross-sectional diagram of B-B in Fig. 3;
Fig. 5 is a partial enlarged schematic diagram of part I in Fig. 3;
Fig. 6 is a schematic structural diagram 1 of a head of a flexible lens sheath according to an embodiment of the present invention;
Fig. 7 is a cross-sectional diagram 1 of A-A in Fig. 6;
Fig. 8 is a cross-sectional diagram 2 of A-A in Fig. 6;
Fig. 9 is a schematic structural diagram 2 of a head of a flexible lens sheath according to an embodiment of the present invention;
Fig. 10 is a schematic diagram showing that the conductive portion is in contact with and conduction with the light source lead according to an embodiment of the present invention;
Fig. 11 is a schematic structural diagram 3 of a portion of a flexible lens sheath according to an embodiment of the present invention;
Fig. 12 is a schematic structural diagram 4 of a portion of a flexible lens sheath according to an embodiment of the present invention;
Fig. 13 is a schematic structural diagram of a sterilization jacket according to an embodiment of the present invention.

### Description of reference numerals:

1-Flexible lens sheath;
11-Endoscope receiving structure;
12-Lens sheath base;
13-Insertion portion;
131-Support portion;
132-Transparent portion;
14-Lens sheath flexible tube;
15-Water and gas valve structure;
16-Bending control structure;
17-Instrument tube receiving structure;
18-Fixing structure;
2-Endoscope body;
21-Endoscope handle;
22-Endoscope flexible tube;
23-Image acquisition package structure;
231-Image acquisition component;
232-Circuit board;
233-Package outer tube;
24-Image acquisition lead;
3-Sterilization jacket;
31-Connection portion;
32-Foldable antibacterial cover;
33-Compression box;
4-Light source component;
5-Conductive component;
6-Light source lead;
7-Power cord;
8-Multi-lumen tube;
81-Flexible tube channel;
82-Instrument tube channel;
83-Light source lead channel.

### DETAILED DESCRIPTION

The technical solutions in the embodiments of the present invention will be clearly and completely described hereafter in combination with the drawings in the embodiments of the present invention. It is apparent that the described embodiments are only a part of the embodiments of the present invention, but not the whole. Based on the embodiments of the present invention, all the other embodiments obtained by those skilled in the art without inventive effort are within the scope of the present invention.

Terms "first", "second", "third", "fourth", and the like (if any) in the specification and claims of the present invention and the foregoing accompanying drawings are used to distinguish similar objects, but do not need to be used for describing a specific sequence or an order. It should be understood that data used in this way can be interchanged under appropriate circumstances, so that the embodiments of the present invention described herein can be implemented in an order other than those illustrated or described herein. In addition, terms "including", "having", and any variations thereof are intended to cover non-exclusive inclusions, for example, processes, methods, systems, products, or devices that contain a series of steps or units need not be limited to those clearly listed steps or units, but may include other steps or units not explicitly listed or inherent to these processes, methods, products, or devices.

The technical solutions of the present invention are described in detail below with reference to the specific embodiments. The following several embodiments may be combined with each other, and a same or similar concept or process may not be described again in some embodiments.

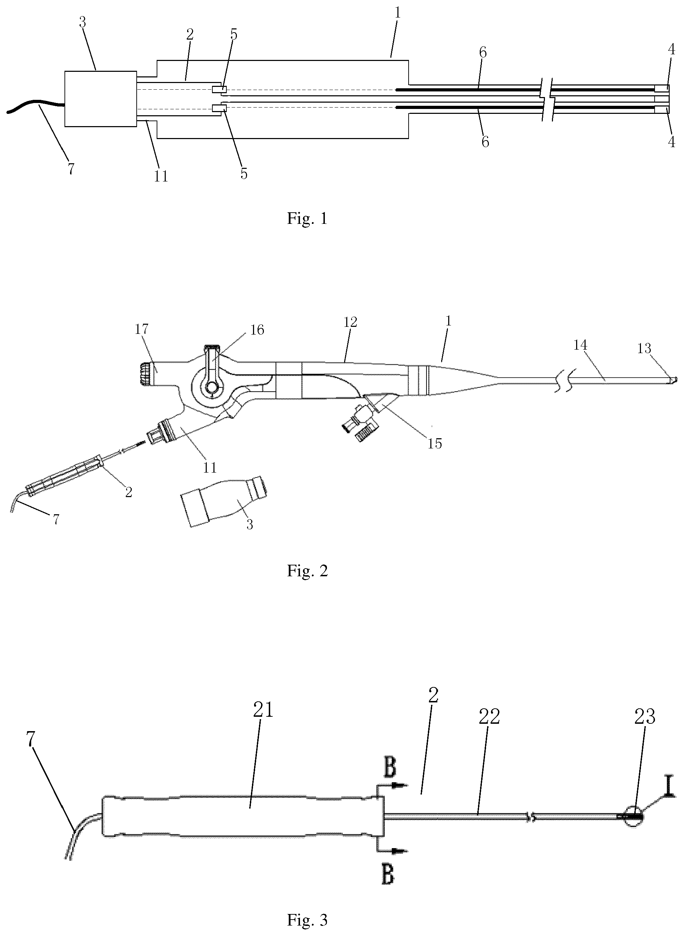

Fig. 1 is a schematic structural diagram 1 of an endoscope device according to an embodiment of the present invention.

With reference to Fig. 1, an endoscope device includes a disposable flexible lens sheath 1, a reusable endoscope body 2, and a disposable sterilization jacket 3, wherein the endoscope body 2 may be received in the flexible lens sheath 1 from an endoscope receiving structure 11 of the flexible lens sheath, and the sterilization jacket 3 is mounted at an entrance of the endoscope receiving structure 11, so that the endoscope body 2 which is received in the flexible lens sheath 1 is isolated from the outside.

The flexible lens sheath 1 may be understood as a lens sheath that may have a bending endoscope tube, wherein the bending endoscope tube has a structure that may be bent in a controlled manner, and any bending structure does not depart from the scope of the present embodiment and its optional solutions.

The endoscope body 2 may be understood as a structure that may achieve endoscopy in the body, and specifically may have an image acquisition component.

The sterilization jacket 3 may be understood as any sheath structure that may be adapted to realize the connection of the endoscope body 2 and cause the endoscope body 2 to be isolated from the outside after the endoscope body 2 is connected to the flexible lens sheath 1. The sterilization jacket 3 and the flexible lens sheath 1 may be assembled together or integrated.

In the above embodiment, with the disposable flexible lens sheath, the reusable endoscope body and the disposable sterilization jacket, the relevant personnel will only come into contact with the disposable flexible lens sheath and sterilization jacket and is completely isolated from the reusable endoscope body when he uses the endoscope device, it may effectively avoid the occurrence of bacterial contamination, reduce the risk of surgical infection, and is convenient, safe and reliable to use. Therefore, it may avoid or reduce the sterilization and disinfection of the endoscope body in the present application, thereby avoiding or reducing the problems caused by the sterilization and disinfection. In addition, since only the flexible lens sheath and the sterilization jacket are disposable, the costs may be reduced.

Meanwhile, in the above embodiment, since the light source component is disposed in the flexible lens sheath, the endoscope body may only supply power to the light source component after being conducted with the lens sheath, which may avoid the astigmatism and reflection caused by the integration of the light source inside the endoscope body as compared with the solution in which the image acquisition component and the light source component are packaged together in the endoscope body.

In the present embodiment, a light source component 4 is provided near a front end of the flexible lens sheath 1, the endoscope body 2 is provided with a conductive component, a power cord 7 is connected to a rear end of the endoscope body 2, and the power cord 7 is conducted with the conductive component 5, the power cord 7 either in indirect connection by wires or components or in direct connection; after the endoscope body 2 is received in the flexible lens sheath 1, the conductive component 5 are directly or indirectly conducted with a light source lead 6 of the light source component 4, so that the light source component 4 is powered, and the light source lead 6 may be directly conductive connection with the conductive component 5, so that further, the conductive component 5 may be contacted conductively with the light source lead 6, wherein the light source lead 6 may also be in indirect conductively connection with the conductive component 5 through other components or wires, and further, the conductive member 5 may be in contact and conduction with the components or wires.

The light source component 4 may be any component that may be powered to emit light, such as an LED component , a light guide fiber component.

In the above embodiment, since the light source component is disposed in the flexible lens sheath, the endoscope body may only supply power to the light source component after being received to the lens sheath, which may avoid the astigmatism and reflection caused by the integration of the light source into the endoscope body as compared with the solution in which the image acquisition component and the light source component are packaged together in the endoscope body.

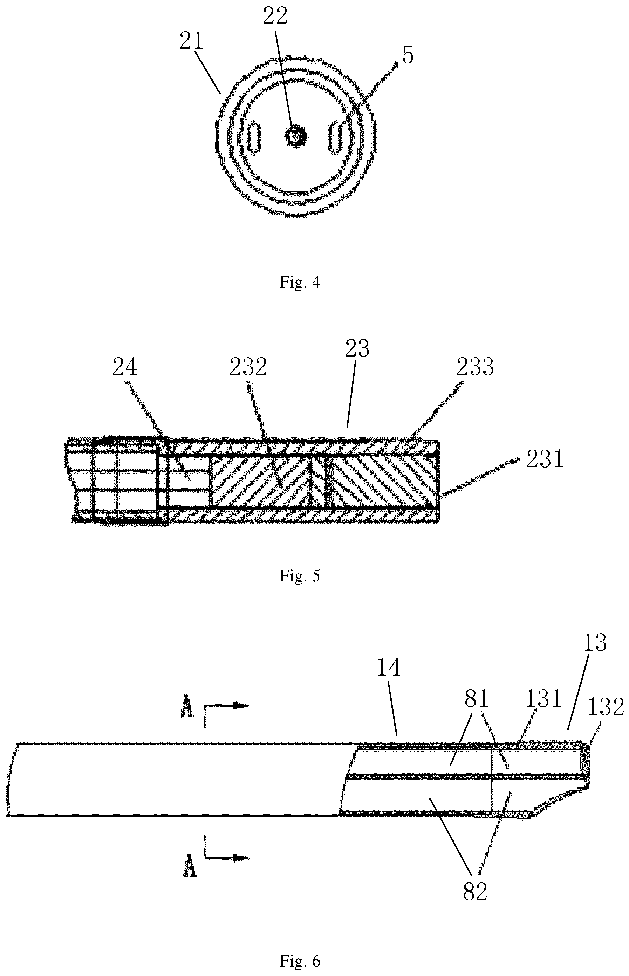

Fig. 2 is a schematic structural diagram 2 of an endoscope device according to an embodiment of the present invention; Fig. 3 is a schematic structural diagram of an endoscope body according to an embodiment of the present invention; Fig. 4 is a cross-section diagram taken from a line B-B in Fig. 3; Fig. 5 is a partial enlarged schematic diagram of part I in Fig. 3.

With reference to Fig. 2 to Fig.5, the endoscope body 2 includes an endoscope handle 21, an endoscope flexible tube 22 connected to the endoscope handle 21 and an image acquisition package structure 23 disposed at a front end of the endoscope flexible tube 22, and the conductive component 5 is disposed at the endoscope handle 21.

In the above embodiment, with the movement of the endoscope handle 21, it is convenient to supply power after plugging in one action. Wherein, the conductive component 5 may be disposed at a front end of the endoscope handle 21 to protrude from a front end surface of the endoscope handle 21, and the conductive component 5 may also be recessed on the front end surface of the endoscope handle 21 or be flush with the front end surface of the endoscope handle 21.

The endoscope handle 21 may be understood as a structure suitable for a person to hold the endoscope body 2 and send it into the flexible lens sheath. The endoscope receiving structure 11 may be disposed on the endoscope handle 21, and a size of a channel in the endoscope receiving structure 11 may be matched with an outer diameter size of the endoscope handle 21, so as to be suitable for guiding the insertion and helping to ensure the stability of the position. The channel in the endoscope receiving structure 11 may be communicated to an internal space of the endoscope handle 21 and further communicated to the flexible tube channel.

The image acquisition package structure 23 may be understood as an entity that has any image acquisition components and is packaged together by a packaging process to form a complete structure. The light source component may not be packaged in the above embodiments compared with the package structure in the related prior art, therefore the structure is simplified. Further, it can be advantageous to achieve a more compact structure, thereby contributing to the effect of not increasing the outer diameter of the endoscope.

In one embodiment, with reference to Fig. 5, the image acquisition package structure 23 includes an image acquisition component 231 and a circuit board 232 that are packaged together, wherein the circuit board 232 is directly or indirectly connected to the power cord 7, for example, directly or indirectly connected to the power cord 7 through an image acquisition lead 24, and the image acquisition component 231 is connected to the circuit board 232.

In actual implementation, the image acquisition component 231 and the circuit board 232 may be packaged together with a package outer tube 233, wherein the package outer tube 233 may be integrated with a wall of the endoscope flexible tube 22, or may also be understood as a part of the wall of the endoscope flexible tube.

In addition, in the above embodiments, the endoscope body 2 may further have positive effects such as fewer interfaces, convenient use, safety and reliability.

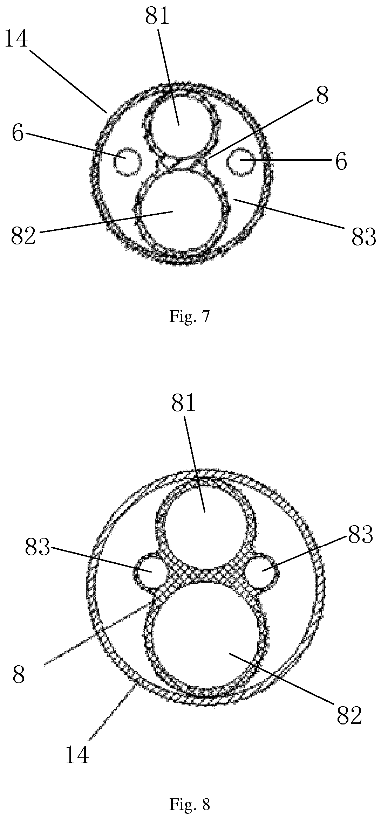

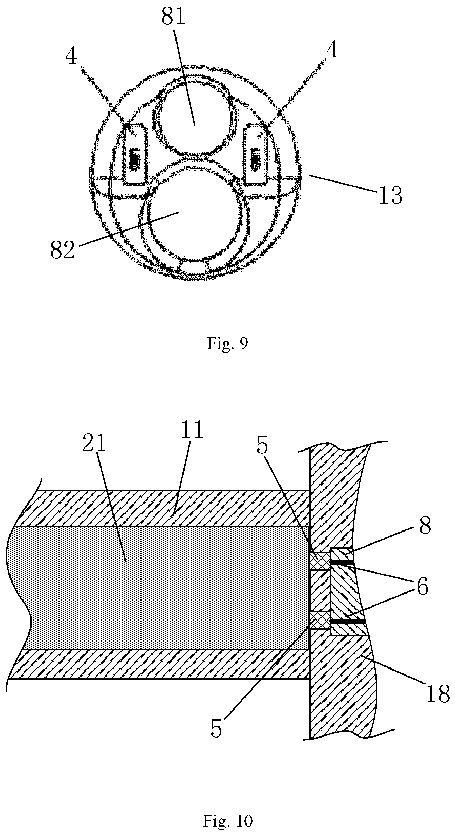

Fig. 6 is a partial structural diagram 1 of a flexible lens sheath according to an embodiment of the present invention; Fig. 7 is a cross-sectional diagram 1 of A-A in Fig. 3; Fig. 8 is a cross-sectional diagram 2 of A-A in Fig. 3; Fig. 9 is a partial schematic structural diagram 2 of a flexible lens sheath according to an embodiment of the present invention.

With reference to Figs. 6 to 9 and Fig. 2, the flexible lens sheath 1 includes an lens sheath base 12, an lens sheath flexible tube 14 and an insertion portion 13, the lens sheath base 12 is connected to a rear end of the lens sheath flexible tube 14, and the insertion portion 13 is disposed at a front end of the lens sheath flexible tube 14.

The endoscope receiving structure 11 is disposed at the lens sheath base 12, a flexible tube channel 81 for receiving the endoscope flexible tube are formed in the lens sheath flexible tube 14 and the insertion portion 13, and the flexible tube channel 81 is directly or indirectly communicated with a channel in the endoscope receiving structure 11.

The sheath base 12 may be understood as a base suitable for disposing the endoscope receiving structure 11, the insertion portion 13 and the sheath flexible tube 14 on it to form the entire base of the flexible lens sheath. In one embodiment, at least one of a water and gas valve structure 15, a bending control structure 16, and an instrument tube receiving structure can be provided on the lens sheath base, i.e.:
the flexible lens sheath 1 is provided with at least one of:
a water and gas valve structure 15;
a bending control structure 16 for controlling the bending of the lens sheath flexible tube 14;
a locking mechanism (not shown) for locking the endoscope body 2 and the flexible lens sheath 1.

Wherein ,the water and gas valve structure 15, the bending control structure 16, and the instrument tube receiving structure 17 may be understood with reference to the water and gas valve structure, the bending control structure and the instrument tube receiving structure of the lens sheath in the related prior art. In other words, the use of any existing water and gas valve structure, bending control structure, and instrument tube receiving structure, or improved water and gas valve structure, bending control structure, and instrument tube receiving structure are used without departing from the above description of the present embodiments.

The lens sheath flexible tube 14 may be understood as a tube structure that may be bent in a controlled manner, and further, the endoscope flexible tube 22 inserted into the endoscope channel 81 may also be bent accordingly.

Further, the lens sheath flexible tube 14 may be disposed at one end of the lens sheath base 12, and the endoscope receiving structure 11 may be disposed at a position close to the other end of the lens sheath base 12 and further may be suitable for realizing the insertion of the endoscope body 2.

The insertion portion 13 may be understood as a structure that is disposed at an end of the lens sheath flexible tube 14 and may be adapted to perform the required operations, wherein the image acquisition component 231 of the image acquisition package structure 23 may be disposed therein. Moreover, the image acquisition package structure 23 may be partially in the flexible tube 14 or may be entirely in the flexible tube 14.

The insertion portion 13 may be or may not be integrated with the lens sheath flexible tube 14. A structure of a head of the insertion portion 13 is optimized, and the head is smaller than the trunk, which facilitates the insertion of the endoscope device into a natural cavity or incision channel of the human body.

In one embodiment, with reference to Fig. 6, the insertion portion 13 may include a support portion 131 and a transparent portion132. The front end of the support portion 131 connected to the transparent portion 132 and a rear end of the support portion 131 connected to the lens sheath flexible tube 14. The image acquisition component 131 may acquire an image in the front via the transparent portion 132.

With reference to Figs. 7 to 9 and Fig. 6, the flexible lens sheath 1 is provided with a multi-lumen tube 8, the multi-lumen tube 8 is provided in the flexible tube channel 81 for receiving the endoscope flexible tube 22, and the flexible tube channel 81 is directly or indirectly communicated with the channel in the endoscope receiving structure 11 to further make the endoscope flexible tube 22 be connected to the flexible tube channel 81 when the endoscope body 2 is inserted into the channel in the endoscope receiving structure 11.

Correspondingly, the flexible lens sheath 1 may also be provided with a related structure for guiding and fixing the endoscope body 2; for example, a track structure may be provided, so that a front end of the track structure is connected to the endoscope receiving structure 11, and a rear end thereof is connected to the lens sheath flexible tube 14 and/or the multi-lumen tube 8. A position between the multi-lumen tube 8 and the lens sheath base 12 may be fixed by a fixing structure, so as to be suitable for the endoscope flexible tube 22 to be inserted into the lens sheath flexible tube 14.

Fig. 10 is a schematic diagram showing that the conductive portion is in contact with and conducted with the light source lead according to an embodiment of the present invention.

Referring to Fig. 10, it illustrates the above-mentioned fixing structure 18 by way of example, wherein one side of the fixing structure 18 may be provided with a first groove for inserting the conductive component 5, and the other side of the fixing structure 18 may be provided with a second groove for inserting the multi-lumen tube 8, so that the conductive component 5 may be connected to the light source lead 6 in the multi-lumen tube 8 after the two sides are inserted respectively.

In other examples, the fixing structure may also adopt other structural forms; for example, a conductive member may be provided therein, so that the conductive component 5 and the light source lead 6 on both sides may be conducted through the conductive member. In addition, it is also possible to achieve the stability of the positions between the components without using the fixing structure, which is suitable for direct or indirect contact and conduction between the conductive component 5 and the light source lead 6 without being limited to the above.

In one embodiment, the light source lead 6 passes through a light source lead channel 83.

In the embodiment shown in Fig. 7, the light source lead channel 83 is disposed outside the multi-lumen tube 8, in particular in a space between the outside of the multi-lumen tube 8 and an inner wall of the lens sheath flexible tube 14, which may realize isolation between the light source lead channel 83 and the flexible tube channel 81.

In the embodiment shown in Fig. 8, the light source lead channel 83 is disposed in the multi-lumen tube 8, and the light source lead channel is isolated from the flexible tube channel.

In the above embodiment, the flexible tube channel corresponding to the image acquisition component may be caused to be not the same as the light source lead channel corresponding to the light source component, with physical separation between the tube and the wall, so as to further effectively prevent or reduce light interference.

In one embodiment, with reference to Figs. 7 to 9, the multi-lumen tube is further provided with an instrument tube channel 82, the instrument tube channel 82 is isolated from the flexible tube channel 81, and the instrument tube channel 82 and the flexible tube channel 81 are distributed in a splayed shape.

Correspondingly, the flexible lens sheath 1 is further provided with an instrument tube receiving structure 17 for receiving an instrument tube, and the instrument tube channel 82 is directly or indirectly communicated with a channel in the instrument tube receiving structure 17.

The splayed shape may be understood as: the cross-sections of the instrument tube channel 82 and the flexible tube channel 81 are both circular, and the centers of the two channels are distributed along a direction of diameter of the lens sheath flexible tube 14. Therefore, the splayed shape may also be understood as a splay-like shape.

The light source component 5 may be disposed on both sides of the splayed shape, i.e., the left and right sides of the splayed shape shown in Fig. 7.

In the above embodiments, it is advantageous to use space more effectively.

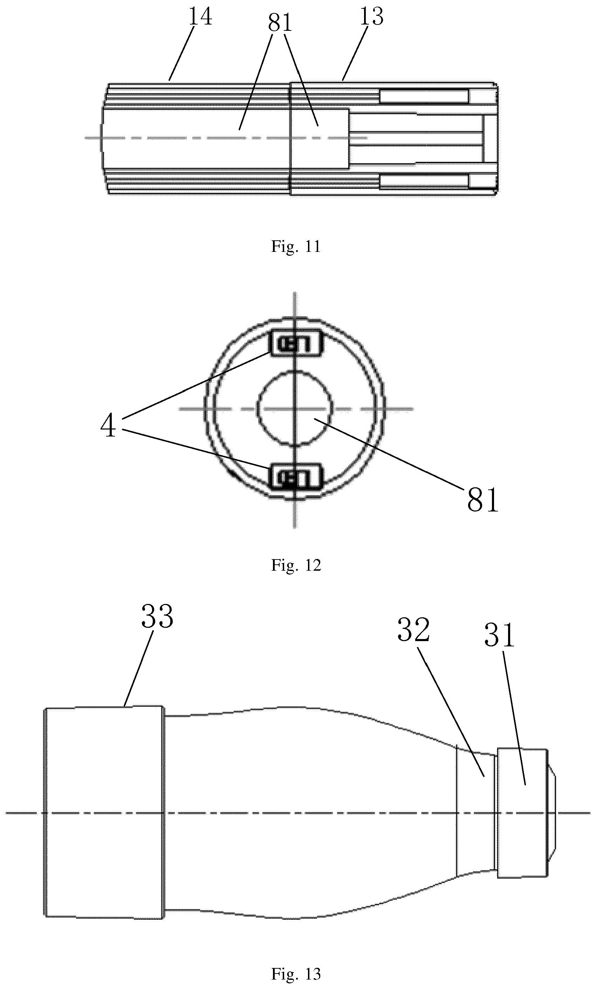

Fig. 11 is a partial structural diagram 3 of a flexible lens sheath according to an embodiment of the present invention; Fig. 12 is a partial schematic structural diagram 4 of a flexible lens sheath according to an embodiment of the present invention.

With reference to Figs. 11 and 12, in one embodiment, it is also possible to have only the flexible tube channel 81 without forming the instrument tube channel 82 and the like. Other structural forms may be as described above.

Fig. 13 is a schematic structural diagram of a sterilization jacket according to an embodiment of the present invention.

With reference to Fig. 13, the sterilization jacket 3 includes a connecting portion 31, a foldable antibacterial cover 32 and a compression box 33; a rear end of the connecting portion 31 is connected to one end of the foldable antibacterial cover 32, and the other end of the foldable antibacterial cover 32 is connected to the compression box 33; the compression box 33 is disposed at a rear end of the foldable antibacterial cover 32, and at least part of the foldable antibacterial cover may be folded in the compression box 33; the other end of the antibacterial cover folded in the compression box is connected to the compression box 33. The antibacterial cover folded in the compression box may be isolated from the rear end of the endoscope body 2 that is connected to the flexible lens sheath 1 after being unfolded.

In the above embodiments, with combination of the sterilization jacket, all the accessible outer surface portions of the patient and the operator are the sterilized and disposable portions, which are completely isolated from the reusable endoscope body, so as to be convenient to use, safe and reliable .

The sterilization jacket 3 includes a foldable sterile cover 32 that may be drawn and folded in the compression box. This solves the problem of bacterial contamination caused by the endoscope body or its connection line for the patient and doctor, and achieves the effect of complete isolation of the area with complete bacteria. During use, after the endoscope body is inserted along the endoscope receiving structure of the flexible lens sheath, the sterilization jacket is directly drawn to isolate the endoscope body and its power cord from the sterile area.

In summary, in the endoscope device provided by the present invention, with the disposable flexible lens sheath, the reusable endoscope body and the disposable sterilization jacket, the relevant personnel will only contact with the disposable flexible lens sheath and sterilization jacket and is completely isolated from the reusable endoscope body when he uses the endoscope device in the present invention, which may effectively avoid the occurrence of bacterial contamination, reduce the risk of surgical infection, and is convenient, safe and reliable to use. Therefore, the present invention may avoid or reduce the process of the sterilization and disinfection of the endoscope body, thereby avoiding or reducing the problems caused by the sterilization and disinfection. In addition, since only the flexible lens sheath and the sterilization jacket are disposable, the costs may be reduced.

And also, in the present invention, since the light source portion is disposed in the flexible lens sheath, the endoscope body may only supply power to the light source portion after being connected to the lens sheath, which may avoid the astigmatism and reflection caused by the integration of the light source inside the endoscope body as compared with the solution in which the image acquisition component and the light source component are packaged together in the endoscope body.

At last, it should be noted that the above various embodiments are only used to describe the technical solutions of the present invention, rather than limiting the technical solutions of the present invention. Even through the present invention is described in detail with reference to the foregoing embodiments, those skilled in the art should understand that they can still modify the technical solutions recorded in the foregoing various embodiments or equivalently replace some or all of the technical features. The invention is defined in the appended claims.

## Claims

1. An endoscope device, comprising a disposable flexible lens sheath(1), a reusable endoscope body(2), and a disposable sterilization jacket(3), wherein the endoscope body(2) can be received in the flexible lens sheath(1) from an endoscope receiving structure(11) of the flexible lens sheath(1), and the sterilization jacket(3) is mounted at an entrance of the endoscope receiving structure(11), so as to isolate the endoscope body(2) received in the flexible lens sheath(1) from the outside;
a light source component(4) is provided near a front end of the flexible lens sheath(1), the endoscope body(2) is provided with a conductive component(5), a rear end of the endoscope body(2) is connected to a power cord(7), and the power cord(7) is conducted with the conductive component(5); after the endoscope body(2) is received in the flexible lens sheath(1), the conductive component(5) is in direct or indirect conduction with a light source lead(6) of the light source component(4), so that the light source component(4) is powered; **characterized in that**:
the sterilization jacket(3) comprises a connecting portion, a foldable antibacterial cover(32) and a compression box(33); a rear end of the connecting portion is connected to one end of the foldable antibacterial cover(32), and the other end of the foldable antibacterial cover(32) is connected to the compression box(33); the compression box(33) is disposed at a rear end of the foldable antibacterial cover(32), and the foldable antibacterial cover(32) may be folded in the compression box(33); the foldable antibacterial sheath may be isolated from the rear end of the endoscope body(2) that is connected to the flexible lens sheath(1) after being unfolded.

2. The endoscope device according to claim 1, wherein the endoscope body(2) comprises an endoscope handle(21), an endoscope flexible tube(22) connected to the endoscope handle(21) and an image acquisition package structure(23) disposed at a front end of the endoscope flexible tube(22), and the conductive component(5) is disposed at the endoscope handle(21).

3. The endoscope device according to claim 2, wherein the image acquisition package structure(23) comprises an image acquisition component(231) and a circuit board(232) that are packaged together, the circuit board(232) is in direct or indirect connection with the power cord(7), and the image acquisition component(231) is connected to the circuit board(232).

4. The endoscope device according to any one of claims 1 to 3, wherein the flexible lens sheath(1) comprises an lens sheath base(12), an lens sheath flexible tube(14) and an insertion portion(13), the lens sheath base(12) is connected to a rear end of the lens sheath flexible tube(14), and the insertion portion(13) is disposed at a front end of the lens sheath flexible tube(14);
the endoscope receiving structure(11) is disposed at the lens sheath base(12), a flexible tube channel(81) for receiving the endoscope flexible tube(22) is formed in the lens sheath flexible tube(14) and the insertion portion(13), and the flexible tube channel(81) is directly or indirectly communicated with a channel in the endoscope receiving structure(11).

5. The endoscope device according to any one of claims 1 to 3, wherein the flexible lens sheath(1) is provided with a multi-lumen tube(8), the multi-lumen tube(8) is provided with the flexible tube channel(81) for receiving the endoscope flexible tube(22), and the flexible tube channel(81) is directly or indirectly communicated with the channel in the endoscope receiving structure(11).

6. The endoscope device according to claim 5, wherein the light source lead(6) passes through a light source lead(6) channel;
the light source lead(6) channel is disposed in the multi-lumen tube(8), and the light source lead(6) channel is isolated from the flexible tube channel(81), or the light source lead(6) channel is disposed outside the multi-lumen tube(8).

7. The endoscope device according to claim 5, wherein the multi-lumen tube(8) is further provided with an instrument tube channel(82), the instrument tube channel(82) is isolated from the flexible tube channel(81), and the instrument tube channel(82) and the flexible tube channel(81) are distributed in a splayed shape.

8. The endoscope device according to claim 7, wherein the flexible lens sheath(1) is further provided with an instrument tube receiving structure(17) for receiving an instrument tube, and the instrument tube channel(82) is directly or indirectly communicated with a channel in the instrument tube receiving structure(17).

9. The endoscope device according to any one of claims 1 to 3, wherein the flexible lens sheath(1) is provided with at least one of:
a water and gas valve structure(15);
a bending control structure(16) for controlling the bending of the lens sheath flexible tube(14);
a locking mechanism for locking the endoscope body(2) and the flexible lens sheath(1).

## Patentansprüche

1. Endoskopgerät, umfassend eine flexible Einweg-Linsenhülle (1), einen wiederverwendbaren Endoskopkörper (2) und eine Einweg-Sterilisationshülle (3), wobei der Endoskopkörper (2) in der flexiblen Linsenhülle (1) von einer Endoskop-Aufnahmestruktur (11) der flexiblen Linsenhülle (1) aufgenommen werden kann, und die Sterilisationshülle (3) an einem Eingang der Endoskop-Aufnahmestruktur (11) angebracht ist, um den in der flexiblen Linsenhülle (1) aufgenommenen Endoskopkörper (2) von außen zu isolieren;
eine Lichtquellenkomponente (4) ist in der Nähe eines vorderen Endes der flexiblen Linsenhülle (1) vorgesehen, der Endoskopkörper (2) ist mit einer leitfähigen Komponente (5) ausgestattet, ein hinteres Ende des Endoskopkörpers (2) ist mit einem Netzkabel (7) verbunden und das Netzkabel (7) ist mit der leitfähigen Komponente (5) verbunden; wenn der Endoskopkörper (2) von der flexiblen Linsenhülle (1) aufgenommen wird, stehen die leitfähigen Komponenten (5) in direkter oder indirekter Verbindung zu einer Lichtquellenleitung (6) der Lichtquellenkomponente (4), so dass die Lichtquellenkomponente (4) mit Strom versorgt wird; **dadurch gekennzeichnet,**
**dass** die Sterilisationshülle (3) einen Verbindungsabschnitt, eine faltbare antibakterielle Abdeckung (32) und eine Kompressionsbox (33) umfasst; ein hinteres Ende des Verbindungsabschnitts mit einem Ende der faltbaren antibakteriellen Abdeckung (32) verbunden ist, und das andere Ende der faltbaren antibakteriellen Abdeckung (32) mit der Kompressionsbox (33) verbunden ist;
die Kompressionsbox (33) ist an einem hinteren Ende der faltbaren antibakteriellen Abdeckung (32) angeordnet und die faltbare antibakterielle Abdeckung (32) kann in die Kompressionsbox (33) gefaltet werden; die faltbare antibakterielle Tasche kann von dem hinteren Ende des Endoskopkörpers (2) isoliert werden, das mit der flexiblen Linsenhülle (1) verbunden ist, sobald sie entfaltet wurde.

2. Das Endoskopgerät nach Anspruch 1, wobei der Endoskopkörper (2) einen Endoskopgriff (21), ein flexibles Endoskoprohr (22), das mit dem Endoskopgriff (21) verbunden ist, und eine Bilderfassungspaket-Struktur (23), die am vorderen Ende des flexiblen Endoskoprohrs (22) angeordnet ist, umfasst und die leitfähigen Komponenten (5) an dem Endoskopgriff (21) angeordnet sind.

3. Das Endoskopgerät nach Anspruch 2, wobei die Bilderfassungspaket-Struktur (23) eine Bilderfassungskomponente (231) und eine Leiterplatte (232) umfasst, die zusammengepackt sind, wobei die Leiterplatte (232) in direkter oder indirekter Verbindung zu dem Netzkabel (7) steht und die Bilderfassungskomponente (231) mit der Leiterplatte (232) verbunden ist.

4. Das Endoskopgerät nach einem der Ansprüche 1 bis 3, wobei die flexible Linsenhülle (1) eine Linsenhüllen-Basis (12), ein flexibles Linsenhüllen-Rohr (14) und ein Einschubteil (13) umfasst, wobei die Linsenhüllen-Basis (12) mit einem hinteren Ende des flexiblen Linsentaschen-Rohrs (14) verbunden ist und das Einschubteil (13) an einem vorderen Ende des flexiblen Linsenhüllen-Rohrs (14) angeordnet ist;
die Endoskop-Aufnahmestruktur (11) ist an der Linsenhüllen-Basis (12) angeordnet, ein flexibler Rohrkanal (81) für das Aufnehmen des flexiblen Endoskop-Rohrs (22) ist in dem flexiblen Linsenhüllen-Rohr (14) und dem Einschubteil (13) geformt, und der flexible Rohrkanal (81) ist direkt oder indirekt mit einem Kanal in der Endoskop-Aufnahmestruktur (11) verbunden.

5. Das Endoskopgerät nach einem der Ansprüche 1 bis 3, wobei die flexible Linsenhülle (1) mit einem Mehrlumenkatheter (8) ausgestattet ist, der Mehrlumenkatheter (8) mit dem flexiblen Rohrkanal (81) ausgestattet ist, um das flexible Endoskoprohr (22) aufzunehmen, und der flexible Rohrkanal (81) direkt oder indirekt mit dem Kanal in der Endoskop-Aufnahmestruktur (11) verbunden ist.

6. Das Endoskopgerät nach Anspruch 5, wobei die Lichtquellenleitung (6) durch den Kanal der Lichtquellenleitung (6) verläuft;
der Kanal der Lichtquellenleitung (6) ist in dem Multilumenkatheter (8) angeordnet und der Kanal der Lichtquellenleitung (6) ist von dem flexiblen Rohrkanal (81) isoliert, oder der Kanal der Lichtquellenleitung (6) ist außerhalb des Multilumenkatheters (8) angeordnet.

7. Das Endoskopgerät nach Anspruch 5, wobei der Multilumenkatheter (8) ferner mit einem Instrumentenrohr-Kanal (82) ausgestattet ist, wobei der Instrumentenrohr-Kanal (82) von dem flexiblen Rohrkanal (81) isoliert ist, und der Instrumentenrohr-Kanal (82) und der flexible Rohrkanal (81) in gespreizter Form verteilt sind.

8. Das Endoskopgerät nach Anspruch 7, wobei die flexible Linsenhülle (1) ferner mit einer Instrumentenrohr-Aufnahmestruktur (17) ausgestattet ist, um ein Instrumentenrohr aufzunehmen, und der Instrumentenrohr-Kanal (82) direkt oder indirekt mit einem Kanal in der Instrumentenrohr-Aufnahmestruktur (17) verbunden ist.

9. Das Endoskopgerät nach einem der Ansprüche 1 bis 3, wobei die flexible Linsenhülle (1) mit mindestens dem Folgenden ausgestattet ist:
Wasser- und Gasventil-Struktur (15);
Biegesteuerungs-Struktur (16) für das Steuern der Biegung des flexiblen Linsenhüllen-Rohrs (14);
Verriegelungsmechanismus für das Verriegeln des Endoskopkörpers (2) und der flexiblen Linsenhülle (1).

## Revendications

1. Dispositif d'endoscope comprenant une gaine de lentille souple jetable (1), un corps d'endoscope réutilisable (2) et une enveloppe de stérilisation jetable (3), dans lequel le corps d'endoscope (2) peut être reçu dans la gaine de lentille souple (1) à partir d'une structure de réception de l'endoscope (11) de la gaine de lentille souple (1) et l'enveloppe de stérilisation (3) étant montée à une entrée de la structure de réception de l'endoscope (11), de manière à isoler de l'extérieur le corps d'endoscope (2) reçu dans la gaine de lentille souple (1) ;
un composant de source lumineuse (4) est prévu près d'une extrémité avant de la gaine de lentille souple (1), le corps d'endoscope (2) est pourvu de composants conducteurs (5), une extrémité arrière du corps d'endoscope (2) est connectée à un cordon d'alimentation (7) et le cordon d'alimentation (7) est conduit avec les composants conducteurs (5) ; une fois que le corps d'endoscope (2) est reçu dans la gaine de lentille souple (1), les composants conducteurs (5) sont en conduction directe ou indirecte avec un conducteur de la source lumineuse (6) du composant de la source lumineuse (4) de manière à ce que le composant de la source lumineuse (4) soit alimenté ; **caractérisé en ce que** :
l'enveloppe de stérilisation (3) comprend une partie de connexion, un couvercle antibactérien pliable (32) et une boîte de compression (33) ; une extrémité arrière de la partie de connexion est connectée à une extrémité du couvercle antibactérien pliable (32) et l'autre extrémité du couvercle antibactérien pliable (32) est connectée à la boîte de compression (33) ;
la boîte de compression (33) est disposée à une extrémité arrière du couvercle antibactérien pliable (32) et le couvercle antibactérien pliable (32) peut être plié dans la boîte de compression (33) ; la gaine antibactérienne pliable peut être isolée de l'extrémité arrière du corps d'endoscope (2), qui est connectée à la gaine de lentille souple (I) après avoir été dépliée.

2. Dispositif d'endoscope selon la revendication 1, dans lequel le corps d'endoscope (2) comprend une poignée d'endoscope (21), un tube d'endoscope souple (22) connecté à la poignée d'endoscope (21) et une structure d'ensemble d'acquisition d'image (23) disposée à une extrémité avant du tube d'endoscope souple (22) et les composants conducteurs (5) sont disposés au niveau de la poignée d'endoscope (21).

3. Dispositif d'endoscope selon la revendication 2, dans lequel la structure d'ensemble d'acquisition d'image (23) comprend un composant d'acquisition d'image (231) et une carte de circuits imprimés (232) qui sont emballés ensemble, la carte de circuits imprimés (232) est en connexion directe ou indirecte avec le cordon d'alimentation (7) et le composant d'acquisition d'image (231) est connecté à la carte de circuits imprimés (232).

4. Dispositif d'endoscope selon l'une quelconque des revendications 1 à 3, dans lequel la gaine de lentille souple (1) comprend une base de la gaine de lentille (12), un tube souple de la gaine de lentille (14) et une partie d'insertion (13), la base de la gaine de lentille (12) est connectée à une extrémité arrière du tube souple de la gaine de lentille (14) et la partie d'insertion (13) est disposée à une extrémité avant du tube souple de la gaine de lentille (14) ;
la structure de réception de l'endoscope (11) est disposée au niveau de la base de la gaine de lentille (12), un canal du tube souple (81) destiné à recevoir le tube souple de l'endoscope (22) est formé dans le tube souple de la gaine de lentille (14) et la partie d'insertion (13) et le canal du tube souple (81) communique directement ou indirectement avec un canal dans la structure de réception de l'endoscope (11).

5. Dispositif d'endoscope selon l'une quelconque des revendications 1 à 3, dans lequel la gaine de lentille souple (1) est pourvue d'un tube à lumières multiples (8), le tube à lumières multiples (8) est pourvu du canal du tube souple (81) destiné à recevoir le tube souple de l'endoscope (22) et le canal du tube souple (81) communique directement ou indirectement avec le canal dans la structure de réception de l'endoscope (11).

6. Dispositif d'endoscope selon la revendication 5, dans lequel le conducteur de la source lumineuse (6) passe à travers le canal du conducteur de la source lumineuse (6) ;
le canal du conducteur de la source lumineuse (6) est disposé dans le tube à lumières multiples (8) et le canal du conducteur de la source lumineuse (6) est isolé du canal du tube souple (81), ou le canal du conducteur de la source lumineuse (6) est disposé à l'extérieur du tube à lumières multiples (8).

7. Dispositif d'endoscope selon la revendication 5, dans lequel le tube à lumières multiples (8) est pourvu en outre d'un canal du tube de l'instrument (82), le canal du tube de l'instrument (82) est isolé du canal du tube souple (81) et le canal du tube de l'instrument (82) et le canal du tube souple (81) sont répartis en une forme évasée.

8. Dispositif d'endoscope selon la revendication 7, dans lequel la gaine de lentille souple (1) est pourvue en outre d'une structure de réception du tube de l'instrument (17) destinée à recevoir un tube de l'instrument et le canal du tube de l'instrument (82) communique directement ou indirectement avec un canal dans la structure de réception du tube de l'instrument (17).

9. Dispositif d'endoscope selon l'une quelconque des revendications 1 à 3, dans lequel la gaine de lentille souple (1) est pourvue d'au moins l'un des éléments suivants :
une structure de vanne d'eau et de gaz (15) ;
une structure de commande de flexion (16) destinée à commander la flexion du tube souple de la gaine de lentille (14) ;
un mécanisme de verrouillage destiné à verrouiller le corps d'endoscope (2) et la gaine de lentille souple (1).
